# EUROPÄISCHE PATENTSCHRIFT

(11) **EP 0 577 981 B1**
(45) Veröffentlichungstag und Bekanntmachung des Hinweises auf die Patenterteilung: **02.09.1998**
(21) Anmeldenummer: 93109057.5
(22) Anmeldetag: 04.06.1993
(51) Int. Cl.: A61C 1/07, A61C 17/00

(54) **Handstückkopf für ein ärztliches oder zahnärztliches Handstück mit einem hin- und herbewegbaren Behandlungswerkzeug**
Head for a medical or dental handpiece comprising an oscillating treatment tool
Tête pour une pièce à main médicale ou dentaire comportant un outil de traitement oscillant

(30) Priorität: 05.06.1992 DE 4218683
(43) Veröffentlichungstag der Anmeldung: 12.01.1994
(73) Patentinhaber: KALTENBACH & VOIGT GMBH & CO., 88400 Biberach (DE)
(72) Erfinder: Strohmaier, Ernst, D-7953 Bad Schussenried (DE)
(74) Vertreter: Schmidt-Evers, Jürgen, Dipl.-Ing.

(56) Entgegenhaltungen:
- WO-A-90/00885
- DE-A- 2 726 325
- FR-A- 2 525 465
- FR-A- 2 574 655
- US-A- 3 552 022

## Beschreibung

Die Erfindung betrifft einen Handstückkopf nach dem Oberbegriff des Anspruchs 1 oder 2. Die Merkmale des Oberbegriffs der Ansprüche 1 bzw. 2 sind aus der WO-A-90/00885 bekannt.

Neben rotierenden Behandlungswerkzeugen gibt es auch hin und her bewegliche, also einen Bewegungshub ausführende Behandlungswerkzeuge, z.B. mit einem flachen bzw. blattförmigen Querschnitt. Bei einem solchen Behandlungswerkszeug kann es sich um ein Werkzeug zum Bearbeiten oder Reinigen der Zähne handeln.

Ein solcher Handstückkopf ist in der US-A- 3 552 022 beschrieben. Bei dieser bekannten Ausgestaltung ist das Behandlungswerkzeug in einer Hubhülse einspannbar, die in Lagern längs verschiebbar im Handstückkopf gelagert ist und durch einen Exenter einer Antriebswelle längs verschiebbar ist, die sich beim vorliegenden Ausführungsbeispiel als Winkelkopf rechtwinklig zur Hubhülse erstreckt. Der Exenter faßt in eine Umfangs-Ringnut der Hubhülse ein. Hierdurch sind die Hubhülse und das Behandlungswerkzeug auch während des Betriebs frei drehbar, und deshalb ist es bei dieser Ausgestaltung lediglich möglich, ganzflächig mit dem vorhandenen, blattförmigen längs verschiebbaren Behandlungswerkzeug, insbesondere eine Flachfeile, zu arbeiten. Sobald die behandelnde Person das Handstück etwas dreht, um ein Drehmoment auf das Behandlungswerkzeug auszuüben, dreht letzteres mit, und deshalb können bestimmte Formbearbeitungen am Zahn nicht vorgenommen werden, eben weil das Behandlungswerkzeug sich immer flächig an die zu bearbeitende Fläche anpaßt.

Würde man eine Antriebsverbindung zum Behandlungswerkzeug schaffen, bei der das Behandlungswerkzeug zwar längs verschiebbar jedoch undrehbar im Handstückkopf gelagert ist, könnte zwar mit dem Handstück ein Drehmoment auf das Behandlungswerkzeug ausgeübt werden und könnten damit auch bestimmte Formarbeiten am Zahn oder zwischen Zähnen ausgeführt werden, jedoch würde dann die Gefahr bestehen, daß mit dem vorgenannten Drehmoment eine beträchtliche Hebelwirkung auf den Zahn ausgeübt und dieser dadurch beschädigt werden könnte. Ein Beispiel hierfür wäre, wenn eine zwischen zwei Zähnen sitzende Flachfeile gedreht werden würde. Die Flachfeile würde aufgrund ihrer Drehung die Zähne spreizen, wobei zu Berücksichtigen ist, daß aufgrund des durch die Länge des Handstücks vorgegebenen aktiven Hebelarms ein beträchliches Drehmoment mit dem Behandlungswerkzeug übertragen werden kann, wodurch Schäden am Zahn hervorgerufen werden können.

Um die vorbeschriebene Schwierigkeit zu vermeiden, ist gemäß WO/90/00885 bei einem Handstückkopf der eingangs angegebenen Art bereits vorgeschlagen worden, das vom Handstück mittels des Behandlungswerkzeugs auf den Zahn übertragbare Drehmoment zu begrenzen. Gemäß Fig. 1, 1A und 2 dieser Druckschrift ist ein winkelförmiger Handstückkopf vorgesehen, wobei das Behandlungswerkszeug einen Befestigungsschaft aufweist, mit dem es von der Bearbeitungsseite des Handstückkopfes her in eine in ihrer Längsrichtung hin und her verschiebbare Hubhülse im Handstückkopf einschiebbar ist, in der der Befestigungsschaft in der eingeschobenen Stellung durch eine formschlüssig wirksame Positionierkupplung positioniert ist.

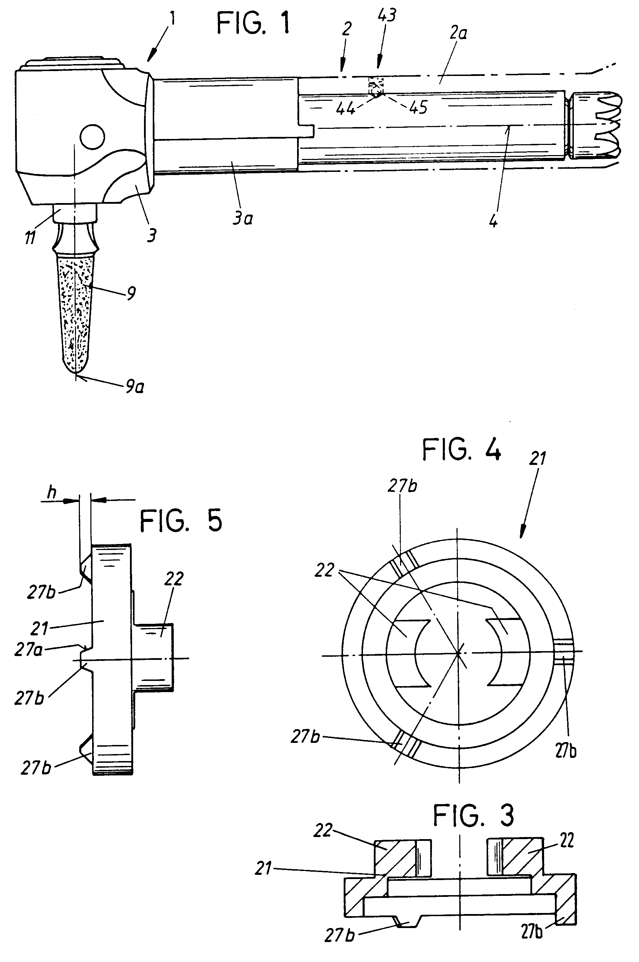

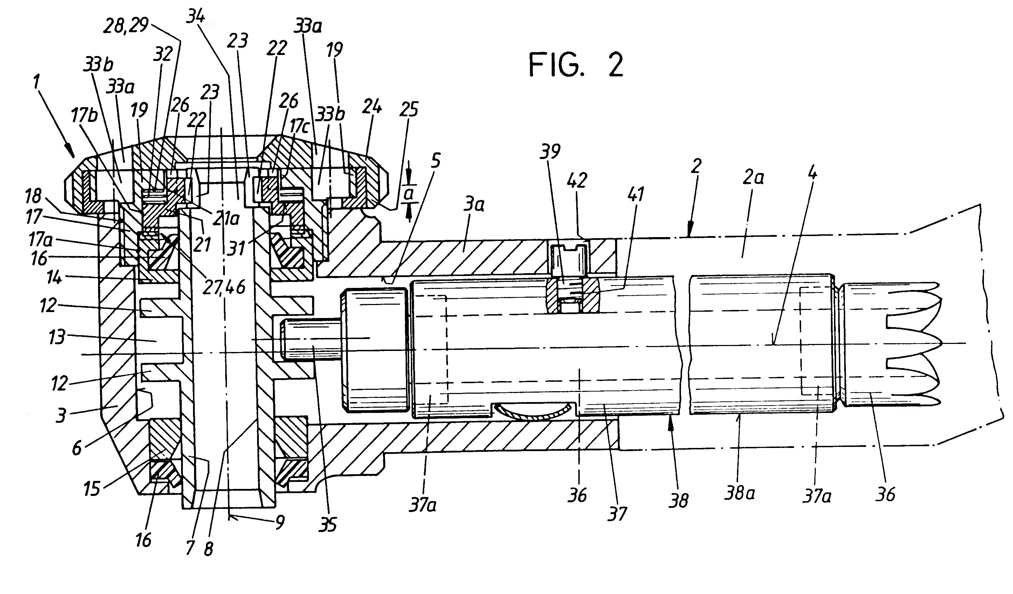

Es sind mehrere Positionierkupplungsstellungen in Form von mehreren, am unteren Rand der Hubhülse vorhandenen Ausnehmungen vorhanden, in die ein Kupplungszapfen am unteren Ende des Befestigungsschaftes des Behandlungswerkzeugs wahlweise durch Einstecken von unten einführbar und durch Abziehen nach unten wiede herausführbar ist. Bei der Ausgestaltung nach Fig. 2 ist diesem bekannten Handstückkopf eine Überdreh-Sicherungsvorrichtung in Form einer Überdrehkupplung zugeordnet, bestehend aus einer oder mehreren auf dem Umfang verteilt angeordneten axialen und rinnenförmigen Ausnehmungen und einem Kupplungskörper in Form einer Kugel, der in einer Ausnehmung in der Innenwandung des Gehäuses so angeordnet ist, daß er in den Innenraum vorragt und somit in eine der rinnenförmigen Kupplungsausnehmungen einfaßt. Die Hubhülse besteht aus einem Kunststoffmaterial mit einer gewissen Flexibilität bzw. Nachgiebigkeit. Wenn mit dem Handstück mittels des Behandlungswerkzeugs ein Drehmoment auf den zu behandelnden Zahn übertragen wird, das größer ist als ein bestimmte Drehmoment, dann geben die zwischen den Kupplungsausnehmungen vorhandenen Stegwände nach, so daß die in vorbeschriebener Weise gebildete Überdrehkupplung überdreht wird. Hierdurch sollen Beschädigungen des Zahns vermieden werden. Diese bekannte Ausgestaltung ist aus mehreren Gründen nachteilig. Es läßt sich das Drehmoment, bei dem die Überdrehkupplung überdrehen soll nicht genau bestimmen, so daß von der Festigkeit des Materials abhängende unterschiedliche Überdrehmomente vorgegeben sind, bei denen Beschädigungen des Zahns bereits stattfinden. Ferner ist damit zu rechnen, daß nach einer Überdrehung sich veränderte Widerstandswerte des Materials ergeben, so daß mit einem Ausleiern der Überdrehkupplung zu rechnen ist. Außerdem läßt sich die Positionierkupplung nur handhabungsunfreundlich bedienen, wobei das Behandlungswerkzeug unmittelbar mit den Fingern ergriffen werden muß, was die Schärfe des Behandlungswerkzeugs beeinträchtigt und auch aus hygienischen Grunden sehr bedenklich ist. Gemäß den Fig. 3 bis 18 ist das Behandlungswerkzeug mit einem Befestigungs- und Antriebsschaft einstückig ausgebildet. Bei einer solchen Ausgestaltung sind die Positionierkupplung und die Überdrehkupplung somit zwischen dem Gehäuse und dem Behandlungswerkzeug direkt wirksam. Dabei sind gemäß Fig. 15 nicht nur die im oberen Bereich des Befestigungsschafts des Behandlungswerkzeugs angeordneten Kupplungsausnehmungen der Positionierkupplung sondern auch eine einzige Kupplungsausnehmung im unteren Bereich des Befestigungsschaftes im Sinne einer Längsrinne ausgeführt, so daß die Längsverschiebbarkeit des Behandlungswerkzeugs gewährleistet ist.

Der Erfindung liegt die Aufgabe zugrunde, einen Handstückkopf nach der in dem Oberbegriff des Anspruchs 1 oder 2 beschriebenen Art so auszugestalten, daß zum einen die Funktionssicherheit mit möglichst konstantem Überdreh-Auslösemoment und/oder zum anderen eine handhabungsfreundliche Dreheinstellung des Behandlungswerkszeugs möglich ist. Außerdem liegt besonderen Ausführungsformen der Erfindung die Aufgabe zugrunde, gewünschte Auslösemomente der Überdrehkupplung und/oder Positionen der Positionierkupplung handhabungsfreundlich variieren zu können und somit den Handstückkopf an verschiedene Behandlungsarten oder Behandlungskritieren anpassen zu können. Dabei soll es im weiteren auch möglich sein, die Überdrehkupplung wahlweise sperren zu können, so daß mit dem Behandlungswerkzeug auch große Drehmomente erzeugt werden können, wenn z.B. die Behandlung dies erfordert.

Die der Erfindung zugrundeliegende Aufgabe wird durch die Merkmale des Anspruchs 1 oder 2 gelöst.

Beim erfindungsgemäßen Handstückkopf nach Anspruch 1 ist eine handhabungsfreundliche Drehposition-Einstellung gewährleistet, wobei das Behandlungswerkzeug vom direkten manuellen Zugriff freigestellt ist und somit eine Veschmutzung und hygienische Beeinträchtigung des Werkzeugs bei der Drehposition-Einstellung unterbleibt. Außerdem ist das Behandlungswerkzeug ohne seine Längsverschiebung und ohne eine Lösung seiner Antriebsverbindung in einer wahlweisen Drehposition einstellbar.

Mit der erfindungsgemäßen Ausgestaltung nach Anspruch 2 wird eine funktionssichere Überdreh-Sicherungsvorrichtung erreicht, bei der die Auslösung der Überdrehkupplung sowohl exakt vorbestimmbar als auch exakt durchführbar ist und zwar auch nach längerer Lebensdauer. Dabei wird nicht nur ein insbesondere blattförmiges Behandlungswerkzeug vor Überlastung geschützt, sondern es werden auch die Zähne vor einer Überlastung geschützt, z.B. dann, wenn ein insbesondere blattförmiges Behandlungswerkzeug durch einen Spalt zwischen zwei Zähnen hindurchgeführt werden muß oder ein im Spalt verklemmtes Behandlungswerkzeug herausgezogen werden muß.

Die Positionierkupplung und die Überdrehkupplung können durch ein und dieselbe Kupplung gebildet sein. Hierdurch wird nicht nur der Herstellungsaufwand sehr verringert, was zu geringeren Herstellungskosten führt, sondern es ist auch der Bedienungsaufwand geringer, und außerdem führt diese Ausgestaltung zu einer kleinen Bauweise, weil nur eine Kupplung vorhanden ist.

Der Handstückkopf keine eine Verstelleinrichtung aufweisen, die eine handhabungsfreundliche Verstellung der Funktionskriterien entweder der Positionierkupplung oder der Überdrehkupplung (soweit vorhanden) oder auch beider Kupplungen ermöglicht. Es läßt sich das Ein- oder Ausrückmoment der jeweiligen Kupplung und somit das Überdrehmoment wahlweise einstellen und an das jeweils vorhandene Behandlungskriterium anpassen. Dabei ist es möglich, die Überdrehkupplung und/oder die Positionierkupplung außer Funktion zu bringen oder die Überdrehkupplung zu verstarren, um z.B. auf das Behandlungswerkzeug unter Berücksichtigung spezieller Behandlungskritierien ein großes Drehinstrument gezielt übertragen zu können.

In den Unteransprüchen sind Merkmale enthalten, die zur Problemlösung beitragen, eine Drehpositionsverstellung in kleinen Drehstufen ermöglichen, zu stabilen und funktionssicheren Ausgestaltungen langer Lebensdauer führen und außerdem einfache und kostengünstig herstellbare sowie kleine Bauweisen vorgeben.

Nachfolgend werden die Erfindung und weitere durch sie erzielbare Vorteile anhand eines bevorzugten Ausführungsbeispiels und einer Zeichnung näher erläutert. Es zeigen
- Fig. 1: einen erfindungsgemäßen Handstückkopf in der Seitenansicht;
- Fig. 2: den Handstückkopf im vertikalen Längsschnitt und in vergrößerter Darstellung;
- Fig. 3: ein Mitnehmerteil des Handstückkopfs im vertikalen Schnitt;
- Fig. 4: das Mitnehmerteil in der Unteransicht;
- Fig. 5: das Mitnehmerteil in der Seitenansicht;
- Fig. 6: den wesentlichen Teil eines abgewandelten Handstückkopfs im vertikalen Längsschnitt und in vergrößerter Darstellung in einer ersten Funktionsstellung;
- Fig. 7: den Handstückkopf nach Fig. 6 in einer zweiten Funktionsstellung;
- Fig. 8: den Teilschnitt VIII-VIII in Fig. 6.

Beim vorliegenden Ausführungsbeispiel ist der Handstückkopf 1 nur ein Teil eines Handstücks 2, das nur teilweise andeutungsweise dargestellt ist. Der Handstückkopf 1 besteht aus einem Winkelkopfgehäuse 3, dessen rückseitige Gehäuseabschnitt 3a im am Handstück 2 montierten Zustand eine vordere Verlängerung der Griffhülse 2a des Handstücks 2 mit einer Längsmittelachse 4 ist.

Wie insbesondere Fig. 2 zeigt, weist das Winkelkopfgehäuse 3 im Bereich seines Gehäuseabschnitts 3a eine axiale Lagerbohrung 5 auf, die nach vorne in eine zu ihr rechtwinklig durchgehend verlaufende und in Stufen gefertigte Lagerbohrung 6 mündet. In der Lagerbohrung 6 ist eine hohlzylindrische Stößelhülse 7 in ihrer Längsrichtung, d.h. quer zum Handstückkopf 1, um ein paar Millimeter hin und her verschiebbar und drehbar gelagert. In der zylindrischen Bohrung 8 der Stößelhülse 7 ist ein in Fig. 1 dargestelltes Bearbeitungswerkzeug 9 mit einem längs seiner Mittelachse 9a angeordneten zylindrischen Halteschaft 11 von der Werkzeugseite her schließend einschiebbar und klemmbar. Das Werkzeug 9 ist von längs oszillierender Arbeitsweise, wobei es sich beim vorliegenden Ausführungsbeispiel um ein im Querschnitt flaches bzw. schwertförmiges Werkzeug 9 im Sinne einer Feile handelt, mit dessen Breit- und/oder Schmalseiten bei seiner Hubbewegung spanabhebend gearbeitet werden kann oder auch - je nach Feinheit - geglättet bzw. poliert werden kann.

Die Stößelhülse 7 weist in ihrem mittleren Bereich zwei einen axialen Abstand voneinander aufweisende Flansche 12 auf, zwischen denen eine Umfangsnut 13 rechteckigen Querschnitts gebildet ist. In ihren zu beiden Seiten der Flansche 12 gelegenen Endbereichen ist die Stößelhülse 7 in Gleitlagerringen 14, 15 gelagert, die im Bereich der zu beiden Seiten vorhandenen Lagerbohrungsabschnitte angeordnet sind, wobei an der Außenseite jedes Gleitlagerringes 14, 15 jeweils ein Dichtungsring 16 mit einer axial nach außen weisenden Abstreiflippe angeordnet ist.

Das werkzeugferne Gleitlager ist mit seinem Gleitlagerring 14 in einer Buchse 17 angeordnet, die in einen äußeren stufenförmig vergrößerten Endabschnitt der Lagerbohrung 6 eingeschraubt ist (Gewinde 18). Die Buchse 17 weist auch eine dreistufige Bohrung auf, mit einem inneren Bohrungsabschnitt 17a, in dem der Gleitlagerring 14 fest eingesetzt ist, einem mittleren Bohrungsabschnitt 17b, in dem ein Mitnehmerring 21 mit Bewegungsspiel drehbar gelagert ist und einem äußeren Bohrungsabschnitt 17c. Der Mitnehmerring 21 weist einen Z-förmigen Querschnitt auf, wobei am äußeren, verjüngten Ringabschnitt 21a innenseitig einander diametral gegenüberliegende Mitnehmerzapfen 22 einstückig angeformt sind, die mit geringem Bewegungsspiel in einander diametral gegenüberliegende radiale Schlitze 23 der Stößelhülse 7 einfassen, wobei die Schlitze 23 axial endseitig ausmünden.

Hierdurch ist eine erste Kupplung zwischen der Stößelhülse 7 und dem Mitnehmerring 21 oder dem Flansch 19 bzw. dem Gehäuse 9 geschaffen, die eine für den Hub erforderliche Längsbewegung der Stößelhülse 7 gestattet, jedoch ein Drehen der Stößelhülse 7 verhindert. Der oder die Kupplungszapfen 22 und die Schlitze 23 können auch umgekehrt angeordnet sein.

Auf dem Flansch 19 ist ein Drehflansch 24 mit geringem Bewegungsspiel drehbar gelagert, der axial außenseitig vom Flansch 19 angeordnet ist und mit seinem äußeren Rand 25 den Flansch 19 C-förmig über- und hintergreift, wodurch der Drehflansch 24 in axialer und radialer Richtung am Flansch 19 drehbar gehalten ist. Am Außenumfang weist der Drehflansch 24 zur Verbesserung einer Griffestigkeit Rillen, Stege oder eine Rändelung auf. In der Innenwandung des Drehflansches 24 oder des Flansches 19 sind einander diametral gegenüberliegend axial und radial angeordnete Schlitze 26 vorgesehen, in die mit geringem Bewegungsspiel die axial vorspringenden Mitnehmerzapfen 22 mit ihren radial äußeren Bereichen einfassen.

Der Gleitlagerring 14 und der Mitnehmerring 21 liegen mit Stirnflächen einander gegenüber, und sie greifen mit jeweils einer an diesen Stirnflächen angeordneten Verzahnung 27 ineinander. Es ist eine solche Verzahnung vorgesehen, die durch Drehen des einen Teils relativ zum anderen Teil überdrückbar ist. Dies ist durch schräge Flanken 27a der Verzahnung 27 möglich, wobei die Neigung der schrägen Flanken (siehe Fig. 5) an das maximale Drehmoment, das die Verzahnung 27 übertragen soll, so angepaßt ist, daß bei Überschreitung dieses Drehmoments der Mitnehmerring 21 axial nach außen aus dem Zahneingriff herausgedrückt wird. Hierzu ist dem Mitnehmerring 21 ein axialer Bewegungsfreiraum zwischen dem Gleitlagerring 14 und der inneren Stufenfläche 28 der Buchse 17 vorgesehen. Dieser Bewegungsfreiraum 29 ist axial durch die Stufenfläche 28 der Buchse 17 und die Stufenfläche 31 des Mitnehmerrings 21 begrenzt. Der Abstand a dazwischen ist größer bemessen, als die Höhe h der Verzahnung 27 und der Höhe von einer oder mehreren im Bewegungsfreiraum 29 angeordneten Wellfedern 32, die den Mitnehmerring 21 axial in den Verzahnungseingriff elastisch vorspannen.

Im Drehflansch 24 und im Flansch 19 sind zwei einander diametral gegenüberliegende, jeweils miteinander in Flucht bringbare axiale Bohrungen 33a, 33b vorgesehen. Durch die äußeren Bohrungen 33a im Drehflansch 24 hindurch können die Zapfen eines nicht dargestellten Schraubschlüssels in die Bohrungen 33b eingesteckt werden zum Zweck des Ein- oder Ausschraubens der Buchse 17. Der Flansch 19 und der Drehflansch 24 sind fest miteinander verbunden und bilden somit ein gemeinsames Drehteil.

Bei der vorliegenden Ausgestaltung weist der Drehflansch 24 ein koaxiales Loch 34 auf, das der Zuführung von Kühl- oder Spülflüssigkeit zum Behandlungswerkzeug durch die Stößelhülse 7 mittels einer nicht dargestellten Zuführungsvorrichtung dienen kann.

Um die axiale Hubbewegung der Stößelhülse 7 zu gewährleisten, bedarf es zu beiden Seiten der Flansche 12 eines Freiraums und einer entsprechenden Länge der Schlitze 23, damit die inneren Mitnehmerzapfen 22 nicht gegen die inneren Schlitzenden stoßen.

Für den Antrieb der Stößelhülse 7 ist am vorderen Ende des Antriebswellenzuges des Handstücks 2 ein exzentrischer zylindrischer Antriebszapfen 35 vorgesehen, der in die Umfangsnut 13 mit geringem Bewegungsspiel einfaßt. Beim vorliegenden Ausführungsbeispiel ist ein den Antriebszapfen 35 tragender Antriebswellenabschnitt 36 mit einer Zahnkupplung oder einem Kegelrad 36 eines Kegelradgetriebes an seinem hinteren Ende vorgesehen, der in einer Lagerhülse 37, vorzugsweise in Gleitlagern 37a drehbar gelagert ist, wodurch eine Antriebspatrone 38 gebildet ist. Die Antriebspatrone 38 sitzt in der Lagerbohrung 5 und ist durch eine darin radial eingeschraubte Fixierschraube 39 gesichert, die in ein radiales Gewindeloch 41 in der Lagerhülse 37 und in ein damit fluchtendes Loch 42 im Gehäuseabschnitt 3a eingeschraubt ist. Die Lagerhülse 37 überragt den Gehäuseabschnitt 3a nach hinten und bildet mit diesem rückseitigen Abschnitt einen Lagerzapfen 38a, der durch eine elastische und beim Herausziehen aus dem Handstück 2 überdrückbare Rastverbindung 43 im Handstück 2 lösbar fixierbar ist. Die Rastverbindung 43 kann durch wenigstens eine Rastausnehmung 44 in der Mantelfläche des Lagerzapfens 38a und eine Rastkugel 45 gebildet sein, die in der Hülse des Handstücks 2 radial verschiebbar gelagert und durch eine Feder in die Rastausnehmung 44 elastisch vorgespannt ist.

Beim vorbeschriebenen Handstückkopf 1 können wahlweise Drehpositions-Einstellungen des Werkzeugs 9 bezüglich des Handstückkopfs 1 eingestellt werden und zwar unabhängig von der jeweiligen Hubstellung der Stößelhülse 7 bzw. deren Antrieb. Die Dreheinstellung kann in kleinen Stufen erfolgen, was durch die verhältnismäßig kleine Teilung der Verzahnung 27 gewährleistet ist. Die Verzahnung 27 bildet eine zweite lösbare Kupplung 46 zwischen der Stößelhülse 7 bzw. dem Mitnehmerring 21 und dem Gleitlagerring 14, die bei Überschreitung eines vorbestimmten Drehmoments selbsttätig öffnet und selbsttätig wieder schließt. Hierdurch ist es möglich, durch Drehen des Drehflansches 24 in die eine oder andere Drehrichtung eine gewünschte Drehposition des Werkzeugs 9 einzustellen. Wenn andererseits am Werkzeug 9 ein vorbestimmtes Drehmoment überschritten wird, folgt die Stößelhülse 7 dieser Drehbeanspruchung selbsttätig, wobei die Verzahnung 27 überdrückt bzw. überdreht wird, d.h., wie ein Klinkensperrtrieb im Leerlauf wirkt. Hierdurch sind Druckbeanspruchungen oder Beschädigungen der Zähne ausgeschlossen, die auf vom Handstück 2 bei, der Handhabung ausgehenden Hebelwirkungen beruhen.

Es ist nicht erforderlich, daß die Verzahnung 27 an beiden Verzahnungsteilen vollständig ausgebildet sein muß. Die Funktion ist auch dann gewährleistet, wenn an einem Teil ein vollständiger Zahnkranz und am anderen Teil theoretisch nur ein, vorzugsweise zwei oder mehrere auf dem Umfang verteilt angeordnete Zähne 27b vorgesehen sind. Bei der vorliegenden Ausgestaltung sind drei Zähne 27b gleichmäßig verteilt angeordnet und zwar vorzugsweise am Mitnehmerring 21, wie es Fig. 4 zeigt. Am Gleitlagerring 14 ist dagegen ein vollständer Zahnkranz mit schräge Flanken aufweisenden entsprechenden Zähnen vorgesehen.

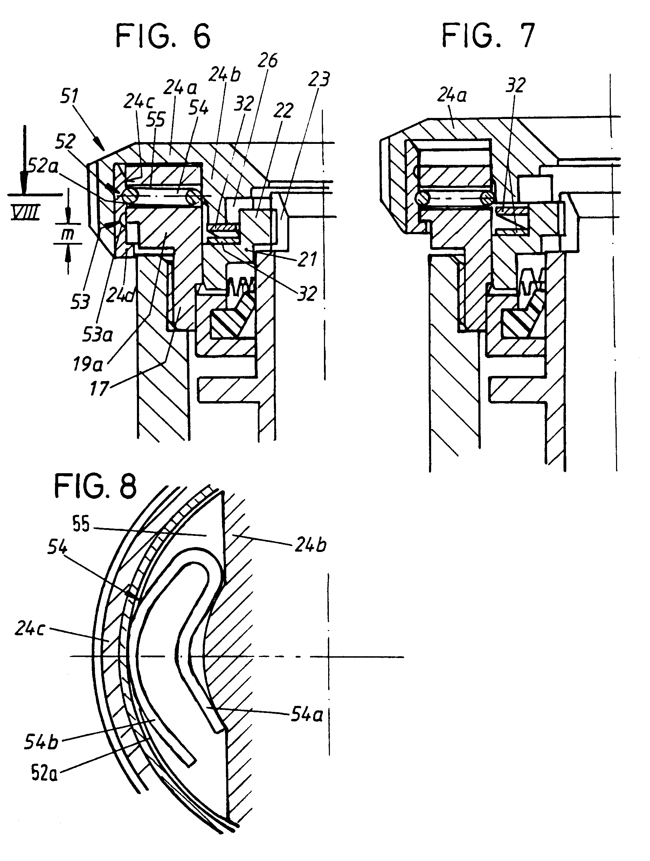

Je nach Art der Zahnbehandlung kann es vorteilhaft sein, mit einem Werkzeug 9 zu arbeiten, daß bis zu einem vorbestimmbaren Drehmoment nicht drehbar ist oder über dieses Drehmoment hinaus nicht drehbar ist oder mit einem Werkzeug zu arbeiten, daß um seine Längsmittelachse leicht oder frei drehbar ist. Dies wird durch die Ausgestaltung gemäß Fig. 6 bis 8 ermöglicht, bei der gleiche oder vergleichbare Teile mit gleichen Bezugszeichen versehen sind, und bei der dem Handstückkopf 1 eine Verstelleinrichtung 51 zugeordnet ist, in deren einen Verstellposition gemäß Fig. 6 die Kupplung 46 eingerückt und somit das Werkzeug 9 begrenzt drehfest ist, und in deren anderer Verstellposition gemäß Fig. 7 die Einrück-Vorspannung der Kupplung 46 verringert oder ganz aufgehoben und somit das Werkzeug 9 leicht oder frei drehbar ist. Es ist auch möglich, der Verstelleinrichtung 51 eine weitere Verstellposition zuzuordnen, in der das Drehmoment, bei dem die Kupplung 46 überdrückbar bzw. überdrehbar ist, sehr groß ist, oder in der Kupplung 46 nicht überdrückbar oder überdrehbar ist.

Die Verstelleinrichtung 51 enthält gemäß Fig. 6 bis 8 ein Verstellglied, mit dem das eine, hier das drehfest mit der Stößelhülse 7 verbundene Kupplungsteil ausrückbar und in der ausgerückten Stellung fixierbar ist (nicht dargestellt), oder mit dem die Einrück-Vorspannung der Kupplung 46 verringert oder ganz aufgehoben werden kann. Bei der vorliegenden Ausgestaltung sind von der die Verstelleinrichtung 51 umfassenden Abwandlung der Flansch 19 sowie der Drehflansch 24 ausgestaltungsmäßig betroffen. Diese Teile sind bei der Ausgestaltung gemäß Fig. 6 bis 8 mit dem Bezugszeichen a ergänzt. Bei dieser Variante weist der Flansch 19a an seinem äußeren Ende keinen Innenflansch auf, sondern seine Innenwandung ist im wesentlichen zylindrisch. Dagegen weist der Drehflansch 24a einen koaxialen inneren Ringvorsprung 24b auf, an dessen inneren Stirnfläche die axiale Feder, hier Wellfedern 32, anliegen, die den Mitnehmerring 21 mit seinen Zähnen in die Einrückstellung der Kupplung vorspannt. Außerdem ist der Drehflansch 24a gegenüber dem Flansch 19a axial verschiebbar, wobei in der eingeschobenen Stellung gemäß Fig. 6 die Wellfedern axial zusammengedrück sind und somit eine Einrück-Vorspannung vorhanden ist, die entsprechend dem Ausführungsbeispiel gemäß Fig. 1 bis 5, überdrückbar ist oder nicht überdrückbar ist. In der Stellung gemäß Fig. 7, in der der Drehflansch 24a gegenüber der Stellung gemäß Fig. 6 axial nach außen verschoben ist, ist die Einrück-Vorspannung der Kupplung, hier die Wellfedern 32, verringert oder entspannt, so daß die Stößelhülse 7 mit dem Werkzeug 9 leicht drehbar ist. Es ist möglich, eine weitere Verstellposition für den Drehflansch 24a vorzusehen, in der er über die Stellung gemäß Fig. 6 hinaus nach innen verschoben ist, und in der die Federn 32 und die Verzahnung 27 soweit zusammengedrückt sind, daß das bewegliche Zahn- bzw. Kupplungsteil 21 nicht auszuweichen vermag und deshalb die Kupplung 46 nicht überdrückbar oder überdrehbar ist.

Die Verschiebbarkeit des Drehflansches 24a auf dem Flansch 19a wird durch ein axiales Schiebelager ermöglicht. Bei der vorliegenden Ausgestaltung übergreift der Drehflansch 24a den Flansch 19a mit einer hohlzylindrischen Ringwand 24c, die um das axiale Verschiebemaß m länger bemessen ist, als der wirksame Flansch 19a der Buchse 17, wobei die Ringwand 24c an ihrem inneren Ende eine Innenschulter 24d aufweist, die durch Anschlag gegen den Flansch 19a die Verschiebung nach außen begrenzt. Der Drehflansch 24 vermag somit mit geringem Bewegungsspiel auf der Umfangsfläche des Flansches 19a axial zu gleiten. Der Verstelleinrichtung 51 ist in der Kupplungsstellung (Fig. 6) und in der quasi Endkupplungsstellung (Fig. 7) jeweils eine elastisch überdrückbare Verrastungsvorrichtung 52, 53 zugeordnet, um eine unbeabsichtigte Verstellung aus der jeweiligen Verstellposition zu vermeiden. Bei der vorliegenden Ausgestaltung ist den beiden Verrastungsvorrichtungen 52, 53 ein gemeinsames Federelement zugeordnet, das am stationären Teil, hier dem Flansch 19a, angeordnet ist und in Rastausnehmungen 52a, 53a in der Innenwandung der Ringwand 24c einzurasten vermag. Bei dem Federelement 54 kann es sich um einen gemäß Fig. 8 U-förmig gebogen und dabei etwas in Umfangsrichtung gekrümmten, vorzugsweise im Querschnitt runden Federdraht handeln, der in einem etwa sekantialen Schlitz 55 im Flansch 19a aufgenommen ist und mit seinem Innenschenkel 54a am inneren Ringvorsprung 24b abgestützt ist und mit seinem konvex gebogenen Außenschenkel 54b in die Rastausnehmungen 52a, 53a einzurasten vermag, die vorzugsweise durch ringförmige, im Querschnitt gerundete Rastnuten in der Innenwandung der Ringwand 24c gebildet sind. Wie bereits beim ersten Ausführungsbeispiel gemäß Fig. 1 bis 5 ist beim zweiten Ausführungsbeispiel gemäß Fig. 6 bis 8 die Innenwandung der hohlzylindrischen Ringwand 24c durch eine dünne Hülse mit der Innenschulter 24d gebildet, die fest in der Ringwand 24c sitzt, z.B. eingepreßt ist.

Die vorbeschriebene Verstelleinrichtung 51 zeichnet sich durch eine einfache, funktionssichere und handhabungsfreundliche Bauweise aus. Für eine Verstellung zwischen der eingerückten Kupplungsstellung gemäß Fig. 6 und der quasi Endkupplungsstellung gemäß Fig. 7 braucht der Benutzer lediglich den Drehflansch 24a an seinem Umfang zu ergreifen und axial in die jeweilige Verstellposition bzw. Verrastungsvorrichtung 52 bzw. 53 zu verschieben. Im übrigen kann der Handstückkopf 1 der Ausgestaltung gemäß Fig. 1 bis 5 entsprechen. Die erfindungsgemäße Verstellvorrichtung 51 ist jedoch nicht auf eine Kupplung 46 gemäß den Fig. 1 bis 5 beschränkt. Im Rahmen der Erfindung ist es möglich, die Verstellvorrichtung 51 auch einer anderen Kupplung zwischen dem Werkzeughalter, hier der Stößelhülse 7, und dem Gehäuse 3 oder einem Anbauteil desselben zuzuordnen.

Der Handstückkopf 1 ist hinsichtlich mehrerer Aspekte der Erfindung vorteilhaft. Ein Aspekt besteht darin, ihn mit einer funktionssicheren Überdreh-Sicherungsvorrichtung auszurüsten, wodurch Beschädigungen des zu bearbeitenden Zahns als auch des Behandlungswerkzeugs 9 vermieden werden können. Bei einer solchen Ausgestaltung des Handstückkopfs 1 kann ein Drehangriffsglied für das Behandlungswerkzeug 9, hier der Drehflansch 24, entfallen, oder es kann auch eine Drehposition-Einstellvorrichtung anderer Ausgestaltung vorgesehen sein.

Ein zweiter Aspekt der Erfindung besteht darin, den Handstückkopf mit einer handhabungsfreundlichen Drehpositions-Einstellvorrichtung für das Behandlungswerkzeug 9 auszurüsten. Bei dieser Ausgestaltung könnte eine Überdreh-Sicherungsvorrichtung entfallen (sofern sie nicht gewünscht ist), oder es könnte auch eine Überdreh-Sicherungsvorrichtung anderer Art vorgesehen sein.

Die Positionierkupplung und die Überdrehkupplung können durch ein und dieselbe Kupplung gebildet sein, wobei die Kupplung von geeigneter Bauweise sein kann.

Weiter kann eine Verstelleinrichtung 51 für die Stellposition-Einstellvorrichtung bzw. Positionierkupplung und/oder die Überdreh-Sicherungsvorrichtung bzw. Überdrehkupplung vorhanden sein. Bei einem solchen erfindungsgemäßen Handstückkopf kann bzw. können die Positionier-Einstellvorrichtung oder die Überdreh-Sicherungsvorrichtung entfallen.

## Patentansprüche

1. Handstückkopf (1) für ein ärztliches oder zahnärztliches Handstück (2),
mit einem insbesondere winkelförmigen Gehäuse (3), in dem ein Behandlungswerkzeug (9) längs seiner Mittelachse (9a) hin und her verschiebbar und drehbar gelagert ist,
wobei dem Handstückkopf (1) eine Drehposition-Einstellvorrichtung für das Behandlungswerkzeug (9) zugeordnet ist, die eine Positionierkupplung (46) mit zwei miteinander zusammenwirkenden Kupplungsteilen (14, 21) aufweist,
**dadurch gekennzeichnet,**
daß am oder im Gehäuse (3) ein von außen manuell zugängliches Drehangriffsglied (24) für das Behandlungswerkzeug (9) bewegbar gelagert ist,
daß die Positionierkupplung (46) im Gehäuse (3) angeordnet und zwischen dem Drehangriffsglied (24) und dem Behandlungswerkzeug (9) wirksam ist, und
daß das Drehangriffsglied (24) und die Positionierkupplung (46) durch eine die Einstellung der Drehposition und die Hubbewegung des Behandlungswerkzeugs (9) ermöglichende Dreh- und Verschiebekupplung (26, 22; 22, 23) mit dem Behandlungswerkzeug (9) verbunden sind.

2. Handstückkopf (1) für ein ärztliches oder zahnärztliches Handstück (2),
mit einem insbesondere winkelförmigen Gehäuse (3), in dem ein Behandlungswerkzeug (9) längs seiner Mittelachse (9a) hin und her verschiebbar und drehbar gelagert ist,
wobei der Handstückkopf (1) eine zwischen dem Gehäuse (3) und dem Behandlungswerkzeug (9) wirksame Überdreh-Sicherungsvorrichtung aufweist, bestehend aus einer Überdrehkupplung (46) mit zwei miteinander zusammenwirkenden und im oder am Gehäuse (3) angeordneten Kupplungsteilen (14, 21), von denen das eine Kupplungsteil (14) mit dem Gehäuse (3) und das andere (21) mit dem Werkzeug (9) verbunden ist, wobei die Überdrehkupplung (46) durch eine die Hubbewegung des Behandlungswerkzeugs (9) ermöglichende Verschiebekupplung (22, 23) mit dem Behandlungswerkzeug (9) verbunden ist,
**dadurch gekennzeichnet,**
daß die Verschiebekupplung (22, 23) zwischen dem mit dem Behandlungswerkzeug (9) verbundenen Kupplungsteil (21) und dem Behandlungswerkzeug (9) angeordnet ist.

3. Handstuckkopf nach Anspruch 1 und 2,
**dadurch gekennzeichnet,**
daß die Positionierkupplung und die Überdrehkupplung durch ein und dieselbe Kupplung (46) gebildet sind und beide Kupplungsteile (14, 21) der gemeinsamen Kupplung (46) Teile des Handstückkopfes (1) sind.

4. Handstückkopf nach einem der vorherigen Ansprüche,
**dadurch gekennzeichnet,**
daß die Positionierkupplung und/oder die Überdrehkupplung (46) in der Einrückstellung (4-6) bei Überschreitung eines vorbestimmten, am Werkzeug (9) wirksamen Drehmoments löst oder überdrückbar ist.

5. Handstückkopf nach einem der vorherigen Ansprüche,
dadurch gekennzeichnet,
daß ein Kupplungsteil (21) in einer Führung bewegbar gelagert und durch eine Federkraft in seine Einrückstellung beaufschlagt ist.

6. Handstückkopf nach einem der vorherigen Ansprüche,
dadurch gekennzeichnet,
daß die Kupplungsteile (14, 21) jeweils ein oder mehrere ineinander greifende Kupplungsausnehmungen (27) und Kupplungszapfen (27b) aufweisen.

7. Handstückkopf nach Anspruch 3,
**dadurch gekennzeichnet,**
daß die vorzugsweise durch eine Verzahnung gebildeten Kupplungsflächen (27a) zwischen den Kupplungsausnehmungen (27) und den Kupplungszapfen (27b) divergent oder schräg angeordnet sind.

8. Handstückkopf nach einem der vorherigen Ansprüche,
**dadurch gekennzeichnet,**
daß die Bewegungsrichtung des bewegbaren Kupplungsteils (21) längs der Mittelachse (9a) des Behandlungswerkzeugs (9) gerichtet ist.

9. Handstückkopf nach einem der vorherigen Ansprüche,
**dadurch gekennzeichnet,**
daß die Positionierkupplung und/oder die Überdrehkupplung (46) konzentrisch zur Mittelachse (9a) des Behandlungswerkzeugs (9) angeordnet sind.

10. Handstückkopf nach einem der vorherigen Ansprüche,
**dadurch gekennzeichnet,**
daß die Kupplungsteile (14, 21) axial nebeneinander angeordnete Ringteile sind, die an ihren einander zugewandten Seiten miteinander in Eingriff bringbar sind und vorzugsweise das Ringteil, das auf der dem Behandlungswerkzeug abgewandten Seite angeordnet ist, das bewegbare Kupplungsteil (21) ist.

11. Handstückkopf nach einem der vorherigen Ansprüche,
**dadurch gekennzeichnet,**
daß das Behandlungswerkzeug (9) in einem Werkzeugaufnahmeteil (7) fixierbar ist, das im Handstückkopf (1) in Richtung der Mittelachse (9a) des Werkzeugs (9) hin und her verschiebbar gelagert und durch die Verschiebekupplung (22, 23; 22, 26) mit dem Werkzeugaufnahmeteil (7) verbunden ist.

12. Handstückkopf nach einem der vorherigen Ansprüche,
**dadurch gekennzeichnet,**
daß ein Kupplungsteil, insbesondere das bewegbare Kupplungsteil (21), durch die Verschiebekupplung (22, 23; 22, 26) mit dem Behandlungswerkzeug (9) oder dem Werkzeugaufnahmeteil (7) verbunden ist.

13. Handstückkopf nach einem der vorherigen Ansprüche,
**dadurch gekennzeichnet,**
daß die Positionierkupplung und/oder die Überdrehkupplung (46) im werkzeugseitigen Endbereich des Werkzeugs (9) oder des Werkzeugaufnahmeteils (7) angeordnet sind.

14. Handstückkopf nach einem der vorherigen Ansprüche,
**dadurch gekennzeichnet,**
daß das Drehangriffsglied (24) von der werkzeugfernen Seite des Handstückkopfs (1) her zugänglich ist, insbesondere an dieser Seite vorzugsweise koaxial zum Werkzeug (9) angeordnet ist.

15. Handstückkopf nach einem der vorherigen Ansprüche,
**dadurch gekennzeichnet,**
daß das Drehangriffsglied (24) durch eine Scheibe, ein Rad oder einen Drehkranz gebildet ist.

16. Handstückkopf nach einem der vorherigen Ansprüche,
**dadurch gekennzeichnet,**
daß ein werkzeugfernes Lager für das Werkzeugaufnahmeteil (7) in einer in den Gehäusekopf (3) einsetzbaren, insbesondere einschraubbaren Buchse (17) angeordnet ist.

17. Handstückkopf nach einem der vorherigen Ansprüche,
**dadurch gekennzeichnet,**
daß das Drehangriffsglied (24) unmittelbar am Gehäusekopf (3) oder an der Buchse (17), vorzugsweise an einem endseitigen Flansch (19) derselben, drehbar gelagert ist.

18. Handstückkopf nach einem der vorherigen Ansprüche,
**dadurch gekennzeichnet,**
daß die Positionierkupplung, die Überdruckkupplung oder die gemeinsame Kupplung (46) zwischen dem Werkzeugaufnahmeteil (7) und dem Gehäusekopf (3) oder der Buchse (17) oder einem Lagerring (14) wirksam ist.

19. Handstuckkopf nach einem der vorhergehenden Ansprüche,
**dadurch gekennzeichnet,**
daß am Gehäuse (3) eine von außen zugängliche Verstelleinrichtung (51) für die Positionierkupplung und/oder die Überdrehkupplung (46) angeordnet ist, mit der die Positionierkupplung und/oder die Überdrehkupplung (46) wahlweise
- in eine Einrückstellung (Fig. 6) und eine Ausrückstellung (Fig. 7) verstellbar ist,
- oder in der Einrückstellung verstarrbar ist,
- oder mit der die Einrück-Vorspannung der Positionierkupplung und/oder Überdrehkupplung (46) vergrößerbar, verringerbar oder ganz aufhebbar ist (Fig. 7).

20. Handstückkopf nach Anspruch 19,
**dadurch gekennzeichnet,**
daß die Verstelleinrichtung (51) von der dem Werkzeug (9) abgewandten Seite her zugänglich ist, insbesondere an dieser Seite vorzugsweise koaxial zur Mittelachse (9a) des Werkzeugs (9) angeordnet ist.

21. Handstückkopf nach Anspruch 19 oder 20,
**dadurch gekennzeichnet,**
daß die Verstelleinrichtung (51) ein axial verschiebbares Verstellglied (24a) aufweist und mit dem zugehörigen Kupplungsteil (21) vorzugsweise zu dessen axialer Verschiebung in Antriebsverbindung steht oder gegen das Kupplungsteil (21) verschiebbar ist.

22. Handstückkopf nach einem der Ansprüche 19 bis 21,
**dadurch gekennzeichnet,**
daß das Verstellglied eine Scheibe ist, die vorzugsweise auf einem in das Gehäuse (3) des Handstückkopfs (1) einschraubbaren Flansch (19a) sitzt und darauf axial verschiebbar ist.

23. Handstückkopf nach Anspruch 21 oder 22,
**dadurch gekennzeichnet,**
daß dem Verstellglied (24a) zur lösbaren Sicherung in seinen Verstellpositionen Sicherungselemente, insbesondere lösbare Rastvorrichtungen (52, 53) zugeordnet sind, vorzugsweise mit in Rastausnehmungen (52a, 53a) einfederndem Federelement (54b).

24. Handstückkopf nach Anspruch 1 und einem der Ansprüche 19 bis 23,
**dadurch gekennzeichnet,**
daß das Drehangriffsglied (24) und das Verstellglied (24a) durch ein und dasselbe Handhabungsteil, insbesondere ein Drehteil, vorzugsweise eine runde Scheibe, gebildet sind.

## Claims

1. Head (1) for a medical or dental handpiece (2), comprising a casing (3), in particular an angled casing, in which a treatment tool (9) is supported so as to be displaceable back and forth along its central axis (9a) and rotatable,
wherein a rotary-position setting device for the treatment tool (9) is assigned to the handpiece head (1), said setting device comprising a positioning coupling (46) with two coupling parts (14, 21) interacting with one another,
**characterised in that**
a rotary-action member (24) for the treatment tool (9) which is manually accessible from outside is movably supported on or in the casing (3),
in that the positioning coupling (46) is disposed in the casing (3) and is effective between the rotary-action member (24) and the treatment tool (9), and
in that the rotary-action member (24) and the positioning coupling (46) are connected to the treatment tool (9) by means of a rotating and sliding coupling (26, 22; 22, 23) enabling the setting of the rotary position and the reciprocating motion of the treatment tool (9).

2. Head (1) for a medical or dental handpiece (2), comprising a casing (3), in particular an angled casing, in which a treatment tool (9) is supported so as to be displaceable back and forth along its central axis (9a) and rotatable,
wherein the handpiece head (1) comprises an overspeed cut-out device which is effective between the casing (3) and the treatment tool (9), consisting of an overspeed coupling (46) with two coupling parts (14, 21) interacting with one another and disposed in or on the casing (3), one coupling part (14) being connected to the casing (3) and the other coupling part (21) being connected to the tool (9), the overspeed coupling (46) being connected to the treatment tool (9) by means of a sliding coupling (22, 23) enabling the reciprocating motion of the treatment tool (9),
**characterised in that**
the sliding coupling (22, 23) is disposed between the coupling part (21) connected to the treatment tool (9) and the treatment tool (9).

3. Handpiece head according to Claims 1 and 2,
**characterised in that**
the positioning coupling and the overspeed coupling are constituted by one and the same coupling (46) and both coupling parts (14, 21) of the common coupling (46) are parts of the handpiece head (1).

4. Handpiece head according to one of the preceding claims,
**characterised in that**
in the event of a predetermined torque acting on the tool (9) being exceeded in the engagement position (4 - 6) the positioning coupling and/or the overspeed coupling (46) is/are released or capable of being subjected to overpressure.

5. Handpiece head according to one of the preceding claims,
**characterised in that**
one coupling part (21) is movably supported in a guide and is pressurised into its engagement position by means of a spring force.

6. Handpiece head according to one of the preceding claims,
**characterised in that**
the coupling parts (14, 21) each comprise one or more coupling recesses (27) and coupling spigots (27b) engaging one another.

7. Handpiece head according to Claim 3,
**characterised in that**
the coupling surfaces (27a) which are preferably constituted by a tooth gearing are disposed divergently or obliquely between the coupling recesses (27) and the coupling spigots (27b).

8. Handpiece head according to one of the preceding claims,
**characterised in that**
the direction of motion of the mobile coupling part (21) is directed along the central axis (9a) of the treatment tool (9).

9. Handpiece head according to one of the preceding claims,
**characterised in that**
the positioning coupling and/or the overspeed coupling (46) are disposed concentrically in relation to the central axis (9a) of the treatment tool (9).

10. Handpiece head according to one of the preceding claims,
**characterised in that**
the coupling parts (14, 21) are axially juxtaposed annular parts which are capable of being brought into engagement with one another on their sides facing one another and the annular part disposed on the side facing away from the treatment tool is preferably the mobile coupling part (21).

11. Handpiece head according to one of the preceding claims,
**characterised in that**
the treatment tool (9) is capable of being fixed in a tool-receiving part (7) which is supported in the handpiece head (1) so as to be displaceable back and forth in the direction of the central axis (9a) of the tool (9) and is connected to the tool-receiving part (7) by means of the sliding coupling (22, 23; 22, 26).

12. Handpiece head according to one of the preceding claims,
**characterised in that**
one coupling part, in particular the mobile coupling part (21), is connected to the treatment tool (9) or to the tool-receiving part (7) by means of the sliding coupling (22, 23; 22, 26).

13. Handpiece head according to one of the preceding claims,
**characterised in that**
the positioning coupling and/or the overspeed coupling (46) are disposed in the end region of the tool (9) or of the tool-receiving part (7) on the tool side.

14. Handpiece head according to one of the preceding claims,
**characterised in that**
the rotary-action member (24) is accessible from the side of the handpiece head (1) remote from the tool, in particular it is disposed on this side preferably coaxially in relation to the tool (9).

15. Handpiece head according to one of the preceding claims,
**characterised in that**
the rotary-action member (24) is constituted by a disc, a wheel or a slewing ring.

16. Handpiece head according to one of the preceding claims,
**characterised in that**
a bearing remote from the tool for the tool-receiving part (7) is disposed in a bush (17) which is capable of being inserted into the casing head (3), in particular is capable of being screwed into the casing head.

17. Handpiece head according to one of the preceding claims,
**characterised in that**
the rotary-action member (24) is rotatably supported directly on the casing head (3) or on the bush (17), preferably on an end flange (19) of said bush.

18. Handpiece head according to one of the preceding claims,
**characterised in that**
the positioning coupling, the overspeed coupling or the common coupling (46) is effective between the tool-receiving part (7) and the casing head (3) or the bush (17) or a bearing ring (14).

19. Handpiece head according to one of the preceding claims,
**characterised in that**
an adjusting device (51) for the positioning coupling and/or the overspeed coupling (46) which is accessible from outside is disposed on the casing (3), with which device the positioning coupling and/or the overspeed coupling (46) is/are alternatively
- adjustable into an engagement position (Fig. 6) and a disengagement position (Fig. 7),
- or capable of being rigidly set in the engagement position,
- or with which device the initial tension of the positioning coupling and/or overspeed coupling (46) is capable of being increased, decreased or nullified entirely (Fig. 7).

20. Handpiece head according to Claim 19,
**characterised in that**
the adjusting device (51) is accessible from the side facing away from the tool (9), in particular it is disposed on this side preferably coaxially in relation to the central axis (9a) of the tool (9).

21. Handpiece head according to Claim 19 or 20,
**characterised in that**
the adjusting device (51) comprises an axially displaceable adjusting member (24a) and is in driving connection with the associated coupling part (21), preferably for the axial displacement thereof, or displaceable towards the coupling part (21).

22. Handpiece head according to one of Claims 19 to 21,
**characterised in that**
the adjusting member is a disc which is preferably seated on a flange (19a) capable of being screwed into the casing (3) of the handpiece head (1) and is axially displaceable thereon.

23. Handpiece head according to Claim 21 or 22,
**characterised in that**
locking elements, in particular releasable latching devices (52, 53), are assigned to the adjusting member (24a) with a view to releasable locking in its positions of adjustment, preferably with a spring element (54b) which is deflected into latching recesses (52a, 53a).

24. Handpiece head according to Claim 1 and one of Claims 19 to 23,
**characterised in that**
the rotary-action member (24) and the adjusting member (24a) are constituted by one and the same operating part, in particular by a rotating part, preferably a circular disc.

## Revendications

1. Tête (1) pour une pièce à main (2) médicale ou dentaire, comprenant un corps (3) en particulier angulaire, dans lequel un outil de traitement (9) est monté mobile en translation en va et vient selon son axe central (9a) et mobile angulairement, un dispositif de réglage de position angulaire pour l'outil de traitement (9) étant associé à la tête (1), ce dispositif comprenant un embrayage de positionnement (46) avec deux parties d'embrayage (14, 21) coopérant l'une avec l'autre,
caractérisée par le fait
qu'un organe d'actionnement angulaire (24) pour l'outil de traitement (9), accessible manuellement de l'extérieur, est monté mobile sur ou dans le corps (3),
que l'embrayage de positionnement (46) est disposé dans le corps (3) et agit entre l'organe d'actionnement angulaire (24) et l'outil de traitement (9), et
que l'organe d'actionnement angulaire (24) et l'embrayage de positionnement (46) sont reliés à l'outil de traitement (9) par un accouplement de rotation et de translation (26, 22 ; 22, 23) permettant le réglage de la position angulaire et le mouvement de translation de l'outil de traitement (9).

2. Tête (1) pour une pièce à main (2) médicale ou dentaire, comprenant un corps (3) en particulier angulaire, dans lequel un outil de traitement (9) est monté mobile en translation en va et vient suivant son axe central (9a) et mobile angulairement, la tête (1) comprenant un dispositif limiteur de couple agissant entre le corps (3) et l'outil de traitement (9) et comprenant un embrayage limiteur de couple (46) avec deux parties d'embrayage (14, 21 ) coopérant l'une avec l'autre et disposées dans ou sur le corps (3), parmi lesquelles une partie d'embrayage (14) est relié au corps (3) et l'autre (21) à l'outil (9), l'embrayage limiteur de couple (46) étant relié à l'outil de traitement (9) par un accouplement de translation (22, 23) permettant le mouvement de translation de l'outil de traitement (9),
caractérisée par le fait
que l'accouplement de translation (22, 23) est disposé entre la partie d'embrayage (21) reliée à l'outil de traitement (9), et l'outil de traitement (9).

3. Tête de pièce à main suivant les revendications 1 et 2, caractérisée par le fait que l'embrayage de positionnement et l'embrayage limiteur de couple sont constitués par un seul et même embrayage (46) et que les deux parties (14, 21) de l'embrayage commun (46) font partie de la tête (1).

4. Tête de pièce à main suivant l'une des revendications précédentes,
caractérisée par le fait
que l'embrayage de positionnement et/ou l'embrayage limiteur de couple (46), dans la position embrayée (4-6), débraye ou patine lorsque le couple agissant sur l'outil (9) dépasse une valeur prédéterminée.

5. Tête de pièce à main suivant l'une des revendications précédentes,
caractérisée par le fait
qu'une partie d'embrayage (21) est montée mobile dans un guide et est sollicitée par une force de ressort dans sa position d'embrayage.

6. Tête de pièce à main suivant l'une des revendications précédentes,
caractérisée par le fait
que les parties d'embrayage (14, 21) présentent respectivement un ou plusieurs creux d'embrayage (24) et tétons d'embrayage (27b) s'interpénétrant les uns les autres.

7. Tête de pièce à main suivant la revendication 3,
caractérisée par le fait
que les surfaces d'embrayage (27a) entre les creux d'embrayage (27) et les tétons d'embrayage (27b), constituées de préférence par une denture, sont disposées en position divergeante ou oblique.

8. Tête de pièce à main suivant l'une des revendications précédentes,
caractérisée par le fait
que la direction de déplacement de la partie d'embrayage mobile (21) est orientée suivant l'axe central (9a) de l'outil de traitement (9).

9. Tête de pièce à main suivant l'une des revendications précédentes,
caractérisée par le fait
que l'embrayage de positionnement et/ou l'embrayage limiteur de couple (46) sont disposés concentriquement par rapport à l'axe central (9a) de l'outil de traitement.

10. Tête de pièce à main suivant l'une des revendications précédentes,
caractérisée par le fait
que les parties d'embrayage (14, 21) sont des parties annulaires disposées axialement côte à côte et pouvant être amenées en prise l'une avec l'autre par leurs côtés tournées l'un vers l'autre et que la partie d'embrayage mobile (21) est constituée de préférence par la partie annulaire disposée sur le côté éloigné de l'outil de traitement.

11. Tête de pièce à main suivant l'une des revendications précédentes,
caractérisée par le fait
que l'outil de traitement (9) petit être fixé dans une partie de réception d'outil (7) qui est montée dans la tête (1) de façon mobile en translation en va et vient suivant l'axe centrale (9a) de l'outil (9) et reliée par l'accouplement de translation (22, 23 ; 22, 26) à la partie de réception d'outil (7).

12. Tête de pièce à main suivant l'une des revendications précédentes,
caractérisée par le fait
qu'une partie d'embrayage, en particulier la partie d'embrayage mobile (21), est reliée par l'accouplement de translation (22, 23 ; 22, 26) à l'outil de traitement (9) ou à la partie de réception d'outil (7).

13. Tête de pièce à main suivant l'une des revendications précédentes,
caractérisée par le fait
que l'embrayage de positionnement et/ou l'embrayage limiteur de couple (46) sont disposés dans la zone d'extrémité, côté outil, de l'outil (9) ou de la partie de réception d'outil (7).

14. Tête de pièce à main suivant l'une des revendications précédentes,
caractérisée par le fait
que l'organe d'actionnement angulaire (4) est accessible depuis le côté de la tête (1), éloigné de l'outil, et est disposé en particulier sur ce côté de préférence co-axialement à l'outil (9).

15. Tête de pièce à main suivant l'une des revendications précédentes,
caractérisée par le fait
que l'organe d'actionnement angulaire (24) est constitué par un disque, une roue ou une couronne de rotation.

16. Tête de pièce à main suivant l'une des revendications précédentes,
caractérisée par le fait
qu'un palier pour la partie de réception d'outil (7), côté éloigné de l'outil, est disposé dans un manchon (17) pouvant être monté, en particulier par vissage, dans le corps de tête (3).

17. Tête de pièce à main suivant l'une des revendications précédentes,
caractérisée par le fait
que l'organe d'actionnement angulaire (24) est monté en rotation directement sur le corps de tête (3) ou sur le manchon (17), de préférence sur une bride (19) prévue côté extrémité sur cette dernière.

18. Tête de pièce à main suivant l'une des revendications précédentes,
caractérisée par le fait
que l'embrayage de positionnement, l'embrayage limiteur de couple ou l'embrayage commun (46) agit entre la partie de réception d'outil (7) et le corps de tête (3) ou le manchon (17) ou une bague de palier (14).

19. Tête de pièce à main suivant l'une des revendications précédentes,
caractérisée par le fait
qu'un dispositif de réglage (51) accessible de l'extérieur est disposé sur la corps (3) pour l'embrayage de positionnement et/ou limiteur de couple (46), dispositif à l'aide duquel l'embrayage de positionnement et/ou l'embrayage limiteur de couple (46), au choix :
- est déplaçable dans une position d'embrayage (figure 6) et une position de débrayage (figure 7),
- ou est verrouillable dans la position d'embrayage,
- ou à l'aide duquel la précontrainte d'embrayage de l'embrayage de positionnement et/ou de l'embrayage limiteur de couple (46) peut être accrue, réduite ou entièrement supprimée (figure 7).

20. Tête de pièce à main suivant la revendication 19,
caractérisée par le fait
que le dispositif de réglage (51) est accessible depuis le côté éloigné de l'outil (9), en étant en particulier disposé sur ce côté de préférence co-axialement à l'axe central (9a) de l'outil (9).

21. Tête de pièce à main suivant la revendications 19 ou 20,
caractérisée par le fait
que le dispositif de réglage (51) présente un organe de réglage (24a) mobile en translation axiale et se trouve en liaison d'entraînement avec la partie d'embrayage (21) associée, de préférence en vue du déplacement en translation axiale de cette dernière, ou est déplaçable en translation contre la partie d'embrayage (21).

22. Tête de pièce à main suivant l'une des revendications 19 à 21,
caractérisée par le fait
que l'organe de réglage est un disque qui est monté de préférence sur une bride (19a) susceptible d'être vissé dans le corps (3) de la tête (1) et est déplaçable en translation axiale sur cette bride.

23. Tête de pièce à main suivant la revendication 21 ou 22,
caractérisée par le fait
que des éléments de sécurité constitués en particulier par des dispositifs d'encliquetage (52, 53) libérables, de préférence avec un élément de ressort (54b) pouvant s'engager élastiquement dans des creux d'encliquetage (52a, 53a), sont associés à l'organe de réglage (24a) en vue de la fixation libérable de ce dernier dans ses positions de réglage.

24. Tête de pièce à main suivant la revendication 1 et l'une des revendications 19 à 23,
caractérisée par le fait
que l'organe d'actionnement angulaire (24) et l'organe de réglage (24a) sont constitués par un même organe de manoeuvre, en particulier un organe de rotation, de préférence un disque circulaire.
